# EUROPEAN PATENT SPECIFICATION

(11) **EP 2 874 545 B1**
(45) Date of publication and mention of the grant of the patent: **15.11.2017**
(21) Application number: 13745604.2
(22) Date of filing: 22.07.2013
(51) Int. Cl.: G01N 33/543, A61B 10/00, G01N 33/48, B01L 3/00, G01N 33/558

(54) **DISPOSABLE TEST DEVICE**
EINWEG-TESTVORRICHTUNG
DISPOSITIF DE TEST JETABLE

(30) Priority: 23.07.2012 EP 12177471
(43) Date of publication of application: 27.05.2015
(73) Proprietor: DML - ABLogics Limited, London SW5 9DB (GB)
(72) Inventor: CATTERUCCIA, Nicoletta, London SW5 9DB (GB); FRIEDLANDER, Uri, London NW3 4AU (GB)
(74) Representative: Nemni, Raffaelo
(86) International application number: PCT/EP2013/065392
(87) International publication number: WO 2014/016236

(56) References cited:
- WO-A1-01/36099
- WO-A2-2009/027935
- WO-A2-2009/036168
- US-A1- 2008 193 926
- US-B2- 7 473 563

## Description

The present invention relates to a disposable test device for detecting a target analyte in a liquid sample derived from biological specimen, e.g. saliva, urine, blood or the like. In particular, the invention relates to test devices containing test strips suited for carrying out lateral flow immunoassays.

### Background of the invention

Immunoassays are widely used to detect a substance of diagnostic interest in a sample by means of the binding of an antibody to its antigen. Monoclonal antibodies are preferred in view of their specificity and selectivity. Immunoassays are routinely used, for example, for the diagnosis of a number of disease including HIV, influenza, hepatitis B or C, autoimmune diseases, neoplasias, etc., as well as for measuring any analyte against which an antibody-antigen reaction may be established. Examples of said analytes include hormones, drugs, markers, proteins, and the like.

Labelled antibodies or antigens are generally used in immunoassays to reveal the presence of a given analyte in the sample. The label is mostly conjugated to an antibody that binds to the analyte. The type of label used may vary, and may include visually detectable labels as well as labels that require instrumentation for detection. Examples of said labels include colloidal gold, enzymes, radioisotopes, fluorescent molecules or other chromophores.

A particularly convenient format for immunoassays is provided by the so-called lateral flow or flow through assays disclosed for instance in US 5,714,389, in WO 01/36099 and in US 5,989,921.

In lateral flow assays, a liquid containing an analyte of interest migrates by capillary force along a membrane strip having suitable reagents impregnated thereon. The liquid sample, applied to one end of the strip, is eluted to a detection zone where a second antibody, immobilized on the strip, allows the visualization of the diagnostic test. Lateral flow assays may be easily carried out in a short time even in non specialized environments and are characterised by high accuracy and sensitivity.

Lateral flow assays may be of the "competitive" or "non-competitive" type. In the competitive-type immunoassays, analyte in a sample is mixed with analyte conjugated to a detectable label. The mixture then migrates along a membrane where the analyte in the sample and the labelled analyte compete for binding to a binding agent specific for the analyte immobilized on the test strip. The amount of labelled analyte detected at the detection zone is inversely proportional to the concentration of analyte in the sample. In "non-competitive", also known as "sandwich"-type immunoassays, the analyte in the sample binds to a first binding reagent conjugated to a label. The sample containing the complex analyte-labelled binding reagent antigen is then contacted with a test strip on which the complex binds to a second binding reagent immobilized on the membrane. In correspondence of the zone where the complex is captured by the second binding reagent, a visual signal appears. Several devices for lateral flow immunoassays have been disclosed. They typically employ different absorbent materials and a membrane, usually a nitrocellulose membrane. Lateral flow devices may be configured so as to have the labelled reagent adsorbed on the test strip so that no further reagent or eluent is required, the liquid sample acting as an eluent. This kind of devices may be convenient when the liquid sample is easy to collect and available in large amounts, for instance in the case of pregnancy tests to be carried out on urine. For other types of assays, an eluent such as a buffer is required. The labelled reagent, instead of being adsorbed on the strip, may also be added to the liquid sample prior to contacting the sample to the test strip.

The assays requiring a separate liquid reagent, such as a buffer system, may be difficult to perform outside an equipped laboratory, by non trained users who are required to measure precise volumes of reagent and or suspend samples to be applied on the test strip. In order to overcome this drawback, a number of devices have been proposed wherein a portion of the test strip is configured so as to form a container or reservoir containing the liquid reagent, with the purpose of avoiding the operations of sampling and measuring the liquid reagent. The known devices are however cumbersome since the containers linked to the test strip are liable to leakage of the fluid contained therein, as a consequence of the geometry intrinsic to the devise hosting the test strip itself.

Most known lateral flow assays have to be directly contacted with the physiological fluid (blood, saliva, urine, etc) and this may be again a potential source of problems for inexpert users.

WO 2009/027935 A2 discloses a chemical sensor cartridge, preferably consisting of two parts, comprising a sample collection element made integral with one part of the cartridge, a sensor element realized by a biosensor chip, a sensor transportation mechanism for transporting sample from the sample collection element to the sensor element, and a connector for linking the sensor element to an external reader device for reading out the sensed parameter.

US 7473563 B2 discloses a sampling and testing device including a sampling member, defining a reservoir that stores an elution solvent, slideably engaged with a base that contains a lateral flow strip adapted to detect specific analytes of interest. During slideable withdrawal of the sampling member from the base, the elution solvent is automatically released to a wick assembly of the sampling member, which is returned to the base after sampling an environment surface for an analyte of interest.

### Description of the invention

An object of the invention is to provide a disposable test device that overcomes the above mentioned drawbacks of known devices and which is simple to use by personnel with no specialized training, has a simple and inexpensive construction and is suitable to make different tests simultaneously on a bodily fluid.

Another object of the invention is to provide a disposable test device allowing an easy sampling of the physiological fluid containing the analyte of interest, increasing thereby the test accuracy and sensitivity, allowing the fluid to be concentrated on a separate member of the device suited also for *in vivo* diagnostic procedures.

These objects are achieved by the disposable test device according to the present invention which comprises the features listed in the appended independent claim 1.

The invention relates also to a method of operation of such a test device, according to claim 13.

Preferred embodiments of the invention are disclosed in the dependent claims.

Essentially, the disposable test device according to the invention comprises: a reagent container, a test chamber and a sample loading devise that can be in the form of a pill.

Tue reagent container is in the form of a cylinder open at one end, wherein a perforated piston of elastic material, such as rubber, can slide. The open end of the container can be closed by a sealing foil to prevent any potential loss of reagent.

The test chamber comprises an outer tubular housing, an inner support member and at least one, preferably a plurality of test strips interposed between the inner support member and the outer housing.

During use, the sample loading pill, which contain absorbent material, previously impregnated with bodily fluid, is sandwiched between the test chamber and the reagent container, from which the sealing foil has been removed, and the bodily fluid mixed with the reagent is transferred to the test strips.

Tue loading pills are particularly convenient for testing analytes contained in the saliva since they may be ingested by the subjects in need of a diagnosis and kept in the mouth for a period of time sufficient to capture the analyte. This test would be particularly convenient, for instance, in the diagnosis of influenza typeA and/or type B infections where the viral antigens are present in minute amounts in the saliva of infected subjects. A variety of clinical condition and infective diseases may also be effectively diagnosed by means of the devices of the invention. Other examples of analytes which may be conveniently detected in the saliva or other physiological fluids (e.g. sweat) include drugs of abuse (cocaine, heroin, amphetamines, barbiturates, benzodiazepines, methadone) and anabolising steroids.

The pills may consist of any suitable absorbent material, typically synthetic, semi-synthetic or natural polymers. Preferred materials include protein matrices (silk, wool) sephadex, cellulose derivatives such as ethylcellulose, hydroxyethylcellulose, hydroxypropylcellulose, in admixtures with lubricants, tabletting agents, surfactants and other conventional excipients.

The size of the pill is not critical and will depend on the specific test for which it has been designed. Anyhow, a length of 10-15 mm and a few mm of diameter will be suited for most applications.

In a preferred embodiment, the sample collection sample loading pill comprises a hollow outer shell molded in soft material such as silicone having an ogival shape, filled with adsorbent material either inert to carry biological samples or chemically derivatized to selectively enrich the analytes to be measured.

Further features of the invention will appear from the following detailed description of purely illustrative and therefore not limitative embodiments thereof made in conjunction with the attached drawings, wherein:
Fig. 1 is a diagrammatic longitudinal sectional view of a disposable test device according to the invention, showing the reagent container, the sample loading pill and the test chamber before assembly;
Fig. 2 is a perspective exploded enlarged view of the reagent container;
Fig. 3 is a perspective view of the test chamber;
Fig. 4 is a longitudinal section through the assembled test chamber;
Figs. 5 a) - f) are diagrammatic sectional views showing successive steps during operation of the device;
Figs. 6 and 7 are diagrammatic perspective exploded views showing two possible shapes of the test chamber;
Figs. 8 to 12 show a different preferred embodiment of the test chamber of the device according to the invention; in particular: Fig. 8 is an exploded diagrammatic perspective view; Fig. 9 is a longitudinal section of the device before assembly; Fig. 10 is a longitudinal section of the device after test has been completed; Fig. 11 is an enlarged plan view showing the test chamber, the sample loading pill and the reagent chamber before assembly; Fig. 12 is a longitudinal section taken along the plane XII-XII of Fig. 11;
Fig. 13 is an enlarged view of the sample loading pill shown in Fig. 8-12; and
Fig. 14 is a sectional view taken along the plane XIV-XIV of Fig. 13.

In the following description similar reference numerals refer to similar parts in the different drawings.

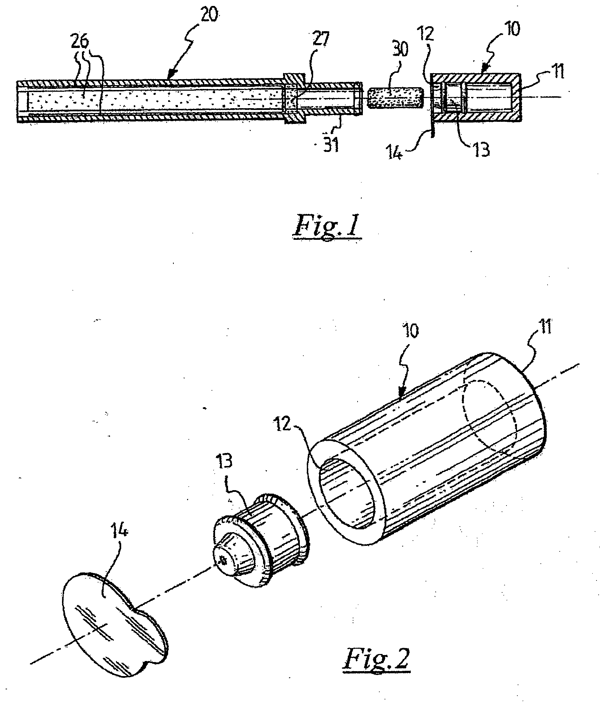

Referring to Fig. 1 of the drawings, the disposable test device according to the invention comprises:
a reagent container 10,
a test chamber 20, and
a sample loading pill 30.

The reagent container 10 (see in particular Fig. 2), preferably in transparent rigid plastic material, is in the form of a cylinder, with a closed end 11 and an open end 12. A rubber piston 13 which seals the container can be inserted through the open end 12 and slide within the cylinder container 10. For filling the container 10 with the reagent, the piston is pushed to the bottom of the container, towards the closed end 11. Then, a needle of a syringe (not shown), which contains the test reagent, is inserted into the rubber piston all the way to the bottom of the container. The syringe is activated and the reagent fills the container 10 while pushing the piston towards the open end 12.

After the container 10 has been filled until the top of the piston is level with open end 12 of the container, the syringe is extracted and the hole made by the syringe needle is hermetically sealed by the elasticity of the rubber piston 13. As a precautionary measure, a sealing foil 14 is heat stacked onto the open end of the container preventing any potential loss of reagent in storage.

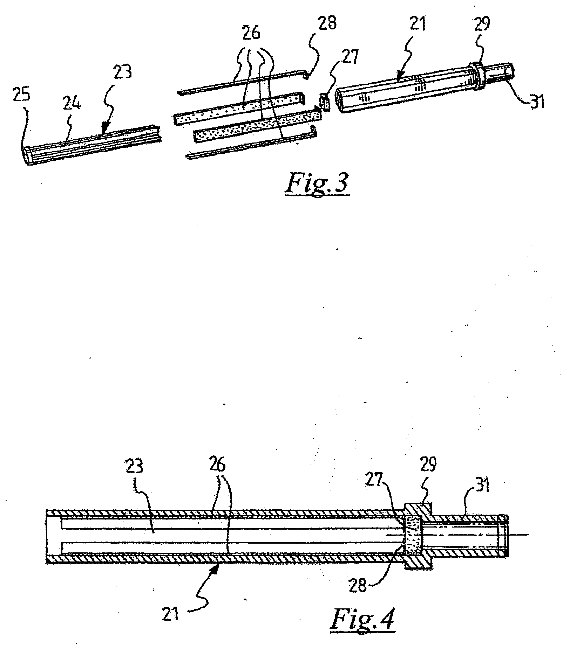

Referring now to Fig. 3, the test chamber 20 is assembled from four main parts:
an outer tubular housing 21 preferably molded in transparent rigid plastic material,
an inner support member 23, also molded in rigid plastic material, having on its outer surface a plurality of longitudinal grooves 24 and a distal radially projecting head 25,
a plurality of test strips 26 able to be housed in said longitudinal grooves 24 such as to be interposed between the inner support member 24 and the outer housing 21, and
an absorbent pad 27 cut or sintered from absorbent material, inserted in the proximal end 29 of the outer housing.

Tue proximal end 28 of the test strips 26 is inwardly folded about 90° such that after being positioned on the inner support member 23 and inserted into the outer housing 21 from the open distal end thereof, they are pushed to form an intimate contact with the absorbent pad 27.

From the proximal end 29 of the outer housing 21 projects a short tubular portion 31, preferably of circular section, having a diameter less than the outer contour of the housing 21 and such as to tightly slide into the reagent container 10.

The sample loading pill 30 is formed of absorbent material which can be used orally or in any other suitable way.

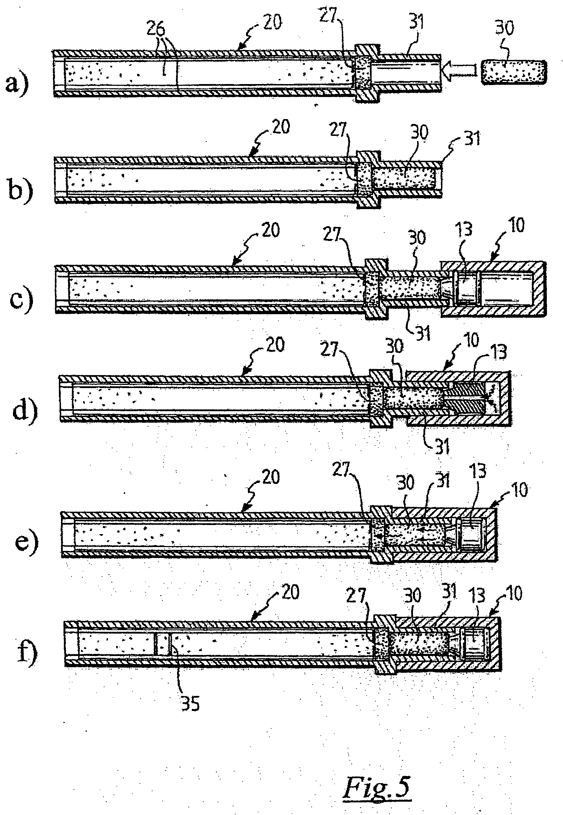

Referring now to Figs. 5 a) to f), the operation of the device will be described.

Tue pill 30 is submerged in the sample and inserted into the tubular portion 31 of the test chamber until it forms an intimate contact with the absorbent pad 27 (Figs. 5 a) and b)).

Thereafter, the sealing foil 14 is removed from the reagent container 10 and the reagent container is mated with the tubular portion 31 of the test chamber (Fig. 5 c)).

The piston 13 inside the reagent container 10 is suitable to be pushed backwards by the tubular portion 31 and the reagent moves towards the sample loading pill 30 through the central hole previously made by the syringe needle in the piston 13 (Fig. 5 d), acting as a valve. In fact, such a hole is hermetically sealed by the
elasticity of the material of the piston 13 until a pressure is applied onto the reagent by the piston. As soon as pressure is applied it opens and let the reagent flow through the "valve".

The reagent pushes through and over the sample loading pill 30 and moves the sample which is impregnated in the sample loading pill towards the absorbent pad 27 (Fig. 5 e)).

The capillary test strips 26 transport the test reagent through them and a detection signal line 35 which is printed on the test strips becomes visible.

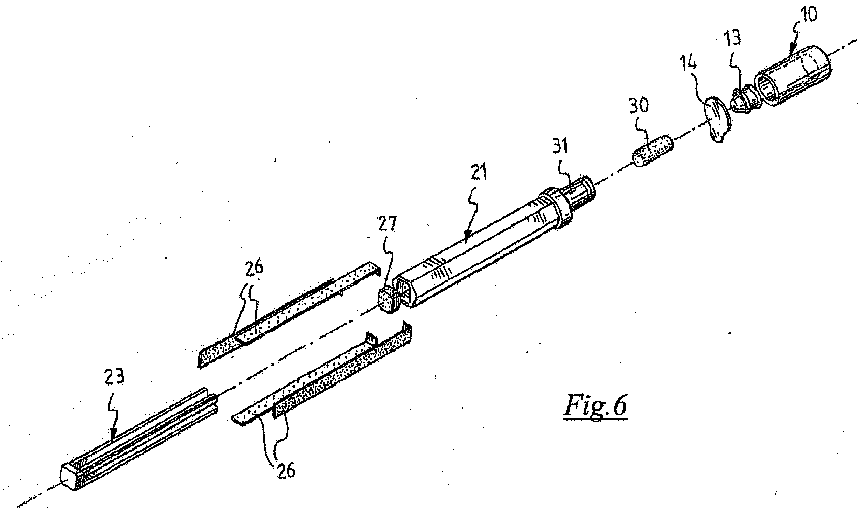

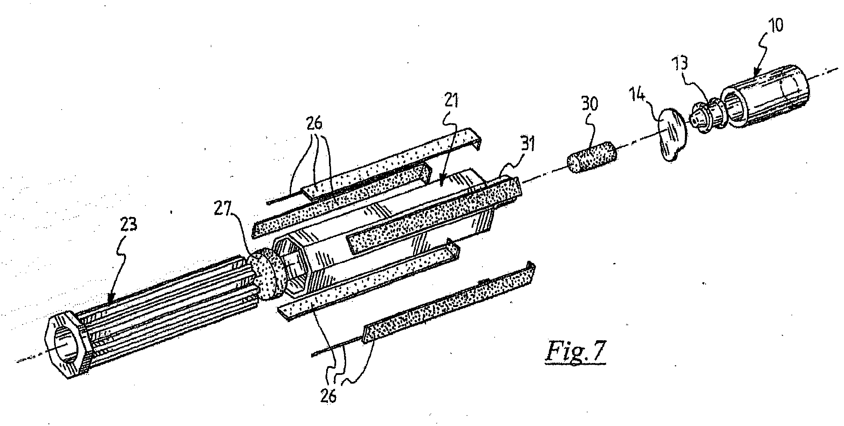

The device of the invention, in particular the test chamber 20 can be of any suitable shape. By way of example only, the embodiments of figures 6 and 7 show the test chamber 20 of square and octagonal cross-section, respectively. Of course the test chamber could also be of substantially circular cross-section.

The reagent chamber also could be of any convenient shape, although it is preferred a circular cross-section for a better sliding of the piston 13 therein.

In the foregoing description, the terms "proximal" and "distal" are referred to the right part of the drawings, i.e. with respect to the reagent chamber 10.

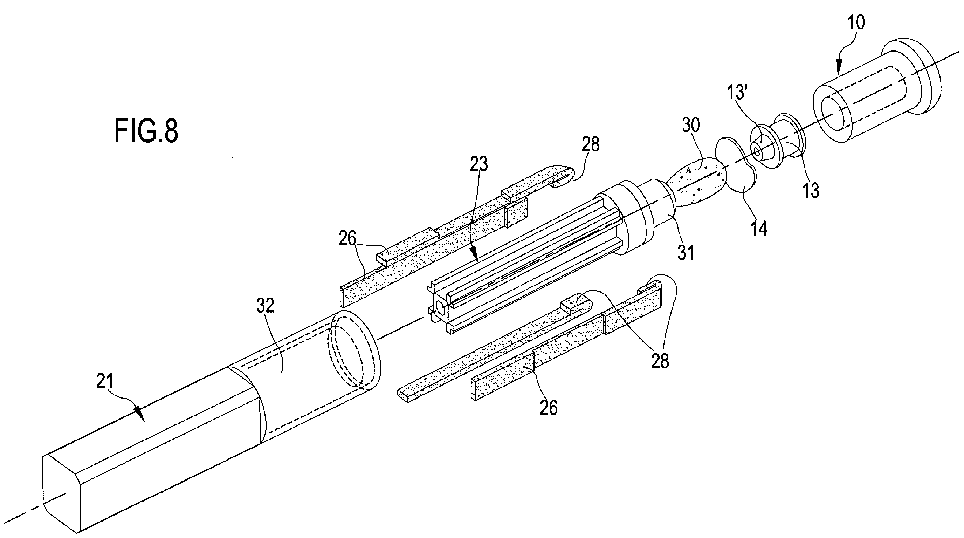

Figures 8 to 12 show the device of the invention with a different preferred embodiment of the test chamber with respect to that shown in particular in Fig. 3. In such figures the same reference numerals as in the previous embodiment are used for indicating the same or similar parts.

According to this embodiment, the tubular portion 31 projects from the proximal end of the inner support member 23 and the proximal end 28 of the test strip 26 is inwardly folded about 180° to be in direct contact with the sample loading pill 30 when the device is assembled, without any extra absorbent pad between the sample loading pill 30 and the test strips 26.

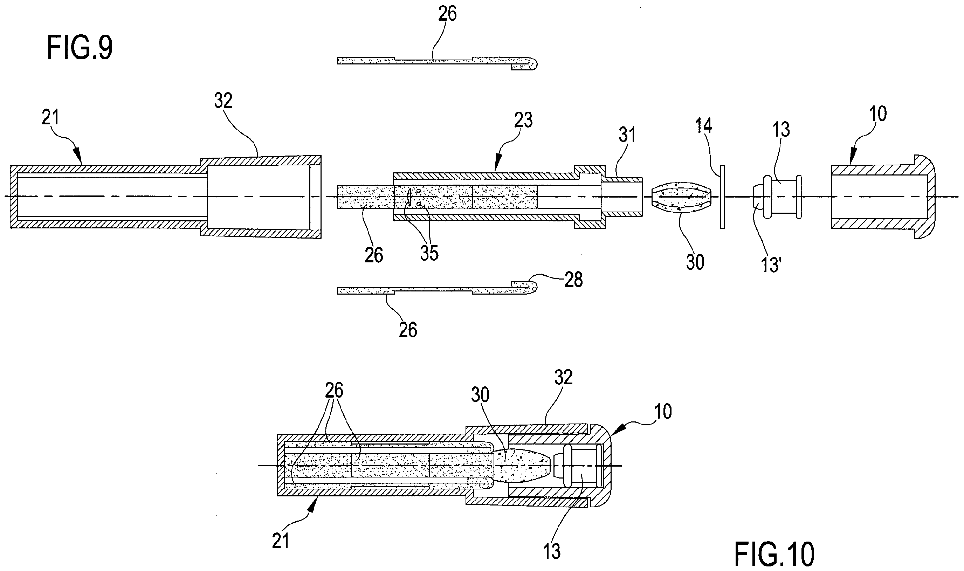

The outer housing 21 has the distal end closed wherein a vent hole is provided and the proximal end opened for insertion of the inner support member 23. An enlarged tubular portion 32 projects from the proximal end of the outer housing 21 to at least partially cover the reagent container 10 when it is inserted on the tubular portion 31 of the inner support member 23 (Fig. 10). In this preferred embodiment the reagent container 1 has in fact an enlarged rear head 10'.

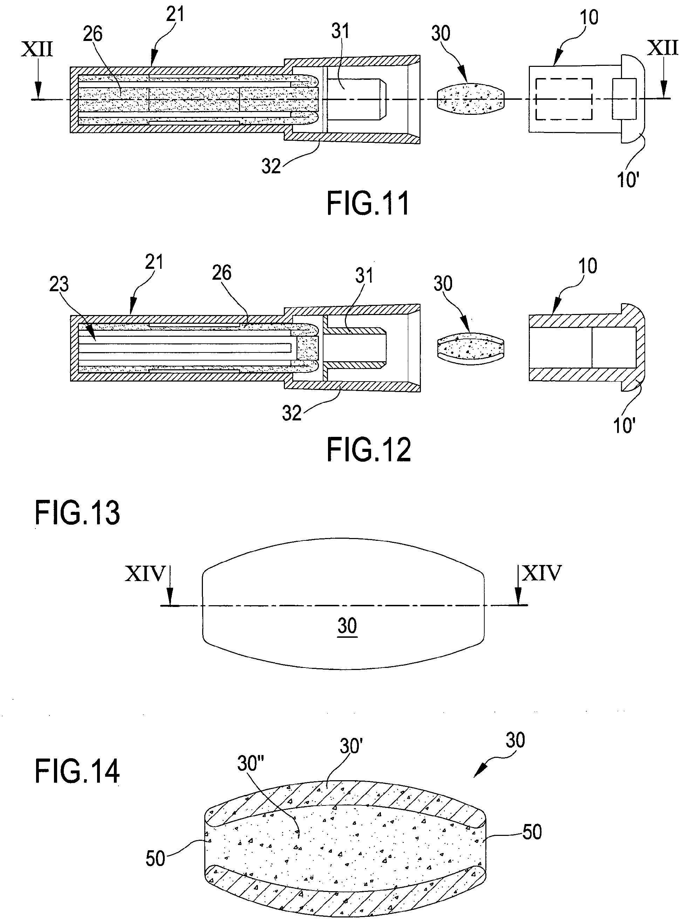

Figures 13, 14 are enlarged views of a preferred form of the sample collection sample loading pill 30 which can be used in all embodiments of the test device according to the present invention. The sample loading pill 30 comprises an outer shell 30' of soft elastic material, e.g. an elastomeric material such as silicone and the like, filled with a liquid retention absorbent material 30" such as nitrocellulose fibers. The outer shell 30' has an ogival shape with two openings 50 at apposite ends aligned along its longitudinal axis, said openings being suitable for collecting the sample to be tested into the absorbent material 30" and for the passage of the reagent through the absorbent material.

For collecting a sample of saliva the sample loading pill is inserted as a whole into the mouth of the patient and by biting and sucking on it for a defined period of time, the absorbent material 30" fills with the sample.

The elasticity of the outer shell 30' allows an easy insertion of the sample loading pill 30 into the tubular portion 31 of the test chamber and when the reagent container 10 is mated with the tubular portion 31, as shown e.g. in Fig. 10, a front conical projection 13' of the piston 13 seals the proximal opening of the outer shell 30' of the sample loading pill so that the reagent can enter through such opening into the absorbent material 30" and exit from the distal opening together with the sample to be tested impregnated into the absorbent material.

The following examples illustrate typical tests which can be carried out with the device according to the invention.

### Example 1

Influenza A And B Test Detection

Influenza test involves the extraction of influenza A and B viral antigen. The specimen is collected by a sterile pill which is kept in the mouth for 5 min. The viral antigens is adsorbed on the sterile pill. The strip is contained in a device cassette.

An antibody specific for the target of interest (anti NP A, anti NPB, HA or NA) is immobilized on a nitrocellulose membrane in the form of a line, called the test line.

A secondary antibody against the primary antibody, generally an anti mouse IgG, is also immobilized on the nitrocellulose membrane in the form of a line called the control line.

Gold nanoparticles conjugated with analytical specific antibody are pre adsorbed into the conjugated pad.

The sample loading pill is then placed onto the housing Gust over the sample pad strip) and after the contact with the specific extraction buffer, the virus particles are disrupted. Through capillary action, the antigens (NPs, HA and NA) are transported into the conjugate pad, where bind gold nanoparticles antibody conjugated (GNP-mAb).

On the test line the anti influenza antibody catches the GNP- mAb coated with antigens, while the second antibody (anti mouse IgG) catches particles which did not bind to an analyte on the control line.

If influenza A or B antigens are present will appear a specific red line together with a positive control line, if influenza antigens are not present will appear just the positive control line.

This immunoassay format described above, can be also applied for a malaria lateral flow test. As the test sample (blood or serum) flows through the membrane the antigen HRP-2, or other specific malaria proteins, bind the gold nanoparticles monoclonal antibody conjugated.

This complex moves further on the membrane to the test region where it is immobilised by the anti HRP-2 (monoclonal) coated on the membrane.

Tue formation of a colored band confirms a positive test result. Tue absence of the colored band in the test region indicates a negative test result. Tue unreacted conjugate move and subsequently immobilised by anti mouse antibodies coated on the membrane at the control region, forming a red band.

### Example 2

### Drug of Abuse Test Detection

Competitive immunoassay: when the target analytes consist of small molecules, as in the case of drugs of abuse, competitive assays are often preferred.

After the extraction of the sample form the pill the analyte are transported into the conjugate pad. Tue conjugate pad contains antibodies that are already bound to an analogue of the target analyte.

If the target analyte is present in the sample it will therefore not bind with the conjugate and will remain unlabelled. As the sample migrates along the membrane and reaches the capture zone an excess of unlabelled analyte will bind to the immobilised antibodies and block the capture of the conjugate, so that no visible line is produced. The unbound conjugate will then bind to the antibodies in the control zone producing a visible control line. A single control line on the membrane is a positive result. Two visible lines in the capture and control zones is a negative result.

Of course the invention is not restricted to the embodiments previously described and shown in the appended drawing, instead many modifications and changes can be made, falling within the scope of protection defined by the appended claims.

## Claims

1. A disposable test device for detecting the presence of an analyte in a liquid sample derived from biological specimen, e.g. saliva, urine, blood or the like, comprising:
- a reagent container (10) containing a reagent,
- a test chamber (20), and
- a sample loading pill (30) of absorbent material suitable to selectively capture and carry the analyte to be tested,
wherein the test chamber contains at least a test strip (26) and has an outer tubular housing (21) and, projecting from the proximal end (29) of the outer housing (21), a tubular portion (31) having a diameter less than the one of the outer contour of the housing (21) to tightly slide into the reagent container (10) the reagent container (10) can be mated with the test chamber (20) with the sample loading pill (30) sandwiched between the reagent container and the test chamber, such that the reagent moves a sample which is impregnated in the sample loading pill towards said at least a test strip (26), **characterized in that**:
- said reagent container (10) is in the form of a cylinder with a closed end (11) and an open end (12) the cylinder containing a perforated piston (13) of elastic material which seals the container itself and a sealing foil (14) heat stacked onto the open end of the container,
- the piston (13) has a central hole previously made by a syringe needle and
- the container (10) is suitable to be inserted on the tubular projection (31) of the test chamber of the device wherein said sample loading pill (30) is housed, the piston being suitable to be pushed backwards by the tubular portion (31) until a pressure is applied onto the reagent solution by sliding the piston onto the device, thereby the reagent moving towards the sample loading pill (30) trough the central hole of the piston acting as a valve.

2. Test device according to claim 1, wherein said test chamber comprises an outer tubular housing (21) and an inner support member (23) having a plurality of longitudinal grooves (24), a plurality of test strips (26) being suitable to be housed in said longitudinal grooves (24) and interposed between the inner support member (23) and the outer housing (21).

3. Test device according to claim 2 , wherein said outer housing (21) has a closed end distal from the reagent container in which a vent hole is provided and the opposite proximal end opened for insertion of the inner support member (23) which is provided with said projection (31) of the test chamber.

4. Test device according to claim 3 , wherein an enlarged tubular portion (32) projects from said proximal end of the outer housing (21) to at least partially cover the reagent container (10) when it is inserted on the tubular portion (31) of the inner support member (23).

5. Test device according to claim 2 , wherein said outer housing (21) has an end distal from the reagent container opened for insertion of the inner support member (23), and the opposite proximal end provided with said projection (31).

6. Test device according to claim 2 , wherein said test strips (26) have their proximal end (28) inwardly folded about 180° to be in intimate contact with said sample loading pill (30).

7. Test device according to claim 2 , wherein said test strips (26) have their proximal end (28) inwardly folded about 90° to be in intimate contact with an absorbent pad (27) in turn in contact with said sample loading pill (30).

8. Test device according to any one of the preceding claims, **characterized in that** said sample loading pill (30) comprises an outer shell (30') of soft elastic material such as silicone, filled with adsorbent material either inert to carry biological samples or chemically derivatized to selectively enrich the analytes to be measured.

9. Test device according to claim 8 , wherein said outer shell (30') has an ogival shape with two openings (50) at opposite ends aligned along its longitudinal axis, said openings being suitable for collecting the sample to be tested into the absorbent material (30") and for the passage of the reagent through the absorbent material.

10. Test device according to any one of the preceding claims, wherein said reagent container (10) and said test chamber (20) are made of transparent, rigid, plastic material.

11. Test device according to any one of the preceding claims, wherein said test chamber (20) has a circular or polygonal cross-section.

12. Test device according to any one of the preceding claims suitable for making different tests simultaneously on the same sample.

13. Method of operation of the test device according to any one of the preceding claims, comprising the following steps:
- insertion of the sample loading pill (30) impregnated with a liquid sample into the tubular projection (31) of the test chamber (20) until it forms an intimate contact with the test strips (26) or the absorbent pad (27);
- removal of the sealing foil (14) from the reagent container (10) and mating the reagent container with the tubular portion (31) of the test chamber (20);
- pushing the piston (13) towards the bottom of the reagent container (10) through the tubular portion (31) so that the reagent moves towards the sample loading pill (30) through a central hole in the piston (13);
- the reagent pushes through and over the sample loading pill (30) and moves the sample which is impregnated in the sample loading pill towards the test strips (26) or the absorbent pad (27) in contact therewith;
- the capillary test strips (26) transport the test reagent through them and a detection signal line (35) which is printed on the test strips becomes visible.

14. Method according to claim 13 , wherein said reagent container (10) is filled with the reagent by pushing the piston (13) to the bottom of the container; inserting a needle of a syringe, which contains the test reagent into the piston (13) all the way to the bottom of the container; activating the syringe so that the reagent fills the container (10) while pushing the piston (13) towards the opening of the container.

## Patentansprüche

1. Ein Einweg-Testgerät zum Nachweis des Vorhandenseins eines Analyten in einer Flüssigkeitsprobe, die aus einem biologischen Untersuchungsgut, wie zum Beispiel Speichel, Urin, Blut oder Ähnlichem, erhalten wurde, die Folgendes umfasst:
- einen Reagens-Behälter (10), der ein Reagens enthält,
- eine Prüfkammer (20), und
- eine Probenträger-Pille (30) aus adsorbierendem Material, die geeignet ist, den zu prüfenden Analyten selektiv zu erfassen und aufzunehmen,
bei welchem die Prüfkammer mindestens einen Teststreifen (26) enthält und ein rohrförmiges Außengehäuse (21) hat und, vom proximalen Ende (29) des Außengehäuses (21) herausragend, ein rohrförmiges Teil (31), dessen Durchmesser kleiner ist als der der Außenkontur des Gehäuses (21), so dass es präzise in den Reagens-Behälter (10) gleitet, der Reagens-Behälter (10) kann an die Prüfkammer (20) angekoppelt werden, wobei die Probenträger-Pille (30) zwischen dem Reagens-Behälter und der Prüfkammer eingebunden ist, so dass das Reagens eine Probe, mit der die Probenträger-Pille (30) getränkt ist, zu genanntem, mindestens einem Teststreifen (26) bewegt, dahingehend gekennzeichnet, dass:
- genannter Reagens-Behälter (10) die Form eines Zylinders mit einem geschlossenen Ende (11) und einem offenen Ende (12) hat, wobei der Zylinder einen perforierten Kolben (13) aus elastischem Material enthält, der den Behälter abdichtet und eine Abdichtungsfolie (14), die mit Wärme auf das offene Ende des Behälters aufgebracht wird,
- der Kolben (13) hat ein zentrales Loch, das zuvor mit einer Spritzennadel angebracht wurde und
- der Behälter (10) ist dazu geeignet, auf den rohrförmigen Vorsprung (31) der Prüfkammer des Geräts aufgesteckt zu werden, in welchem genannte Probenträger-Pille (30) untergebracht ist, wobei der Kolben geeignet ist, dass er von dem rohrförmigen Teil (31) nach hinten geschoben wird, bis, durch das Schieben des Kolbens auf das Gerät, ein Druck auf die Reagens-Lösung ausgeübt wird, wodurch sich das Reagens durch das zentrale, als Ventil wirkende Loch des Kolbens zur Probenträger-Pille (30) bewegt.

2. Prüfgerät nach Anspruch 1, bei welchem genannte Prüfkammer ein rohrförmiges Außengehäuse (21) und ein inneres Führungsstück (23) umfasst, das eine Vielzahl von längs verlaufenden Rillen (24) aufweist und eine Vielzahl von Teststreifen (26), die dazu geeignet sind, von den genannten längs verlaufenden Rillen (24) aufgenommen zu werden, und die zwischengeschoben sind zwischen das innere Führungsstück (23) und das Außengehäuse (21).

3. Prüfgerät nach Anspruch 2, bei welchem genanntes Außengehäuse (21) ein geschlossenes Ende hat, das distal zum Reagens-Behälter gelegen ist und an dem ein Entlüftungsloch angebracht ist und das gegenüberliegende proximale Ende geöffnet ist für das Einführen des inneren Führungsstücks (23), das mit genanntem Vorsprung (31) der Prüfkammer versehen ist.

4. Prüfgerät nach Anspruch 3, bei welchem ein vergrößertes rohrförmiges Teil (32) von genanntem proximalen Ende des Außengehäuses (21) herausragt, um den Reagens-Behälter (10) mindestens teilweise zu überdecken, wenn er auf das rohrförmige Teil (31) des inneren Führungsstücks (23) aufgesteckt ist.

5. Prüfgerät nach Anspruch 2, bei welchem genanntes Außengehäuse (21) ein zum Reagens-Behälter distales Ende hat, das zum Einführen des inneren Führungsstücks (23) geöffnet ist, und bei dem das gegenüberliegende proximale Ende mit genanntem Vorsprung (31) ausgestattet ist.

6. Prüfgerät nach Anspruch 2, bei welchem das proximale Ende (28) der genannten Teststreifen (26) um 180° nach innen gefaltet ist, um in unmittelbarem Kontakt zu genannter Probenträger-Pille (30) zu stehen.

7. Prüfgerät nach Anspruch 2, bei welchem das proximale Ende (28) der genannten Teststreifen (26) um 90° nach innen gefaltet ist, um in unmittelbarem Kontakt zu einer adsorbierenden Kompresse (27) zu stehen, die wiederum mit genannter Probenträger-Pille (30) in Kontakt ist.

8. Prüfgerät nach irgendeinem der vorhergehenden Ansprüche, dahingehend gekennzeichnet, dass genannte Probenträger-Pille (30) eine Außenhaut (30') aus weichem, elastischem Material wie etwa Silikon umfasst, gefüllt mit adsorbierendem Material, entweder inert, um biologische Proben aufzunehmen oder chemisch derivatisiert , um die zu messenden Analyten selektiv anzureichern.

9. Prüfgerät nach Anspruch 8, bei welchem genannte Außenhaut (30') einen spitzbogigen Umriss hat mit zwei, an den gegenüber liegenden Enden befindlichen Öffnungen (50), die entlang ihrer Longitudinalachse ausgerichtet sind, wobei genannte Öffnungen geeignet sind, sowohl die zu prüfende Probe in das adsorbierende Material (30") zu holen, als auch für den Durchfluss des Reagens durch das adsorbierende Material.

10. Prüfgerät nach irgendeinem der vorhergehenden Ansprüche, bei welchem genannter Reagens-Behälter (10) und genannte Prüfkammer (20) aus durchsichtigem, steifem Plastikmaterial gefertigt sind.

11. Prüfgerät nach irgendeinem der vorhergehenden Ansprüche, bei welchem genannte Prüfkammer (20) einen kreisförmigen oder polygonalen Querschnitt hat.

12. Prüfgerät nach irgendeinem der vorhergehenden Ansprüche, das dazu geeignet ist, an derselben Probe gleichzeitig verschiedene Tests auszuführen.

13. Verfahrensweise für den Betrieb des Prüfgeräts nach irgendeinem der vorhergehenden Ansprüche, die folgende Schritte umfasst:
- Einführen der, mit einer flüssigen Probe getränkten, Probenträger-Pille (30) in den rohrförmigen Vorsprung (31) der Prüfkammer (20), bis sie in unmittelbarem Kontakt mit den Teststreifen (26) oder der adsorbierenden Kompresse (27) steht;
- Entfernen der Abdichtungsfolie (14) vom Reagens-Behälter (10) und Ankoppeln des Reagens-Behälters an den rohrförmigen Teil (31) der Prüfkammer (20);
- Schieben des Kolbens (13) in Richtung des Endes des Reagens-Behälters (10) durch das rohrförmige Teil (31), so dass sich das Reagens durch ein zentrales Loch im Kolben (13) zur Probenträger-Pille (30) bewegt;
- das Reagens drückt durch und über die Probenträger-Pille (30) und bewegt die Probe, mit der die Probenträger-Pille getränkt ist, zu den Teststreifen (26) oder zur adsorbierenden Kompresse (27), die damit in Kontakt stehen;
- die kapillaren Teststreifen (26) transportieren das Test-Reagens durch sich hindurch und es wird eine Nachweis-Signallinie, die auf die Teststreifen gedruckt ist, sichtbar.

14. Verfahren nach Anspruch 13, bei welchem genannter Reagens-Behälter (10) mit dem Reagens gefüllt wird, indem der Kolben (13) zum Ende des Behälters geschoben wird; bei welchem eine Spritzennadel, die das Test-Reagens enthält, in den Kolben (13) bis ganz zum Ende des Behälters eingeführt wird; bei welchem die Spritze betätigt wird, so dass das Reagens den Behälter (10) füllt, während es den Kolben (13) in Richtung der Öffnung des Behälters drückt.

## Revendications

1. Dispositif de test jetable pour détecter la présence d'un analyte dans un échantillon liquide obtenu à partir d'un spécimen biologique, par exemple de la salive, de l'urine, du sang ou analogue, comprenant:
- un récipient de réactif (10) contenant un réactif,
- une chambre de test (20), et
- une pilule de chargement d'échantillon (30) en matériau absorbant apte à capturer sélectivement et à transporter l'analyte à tester,
dans lequel la chambre de test contient au moins une bande de test (26) et possède un boîtier tubulaire externe (21) et, dépassant de l'extrémité proximale (29) du boîtier externe (21), une portion tubulaire (31) ayant un diamètre inférieur à celui du contour extérieur du boîtier (21) de façon à coulisser hermétiquement dans le récipient de réactif (10), le récipient de réactif (10) pouvant être couplé à la chambre de test (20) avec la pilule de chargement d'échantillon (30) placée entre le récipient de réactif et la chambre de test, de telle sorte que le réactif déplace un échantillon qui est imprégné dans la pilule de chargement d'échantillon vers la bande de test (26), avec pour caractéristiques que:
- ledit récipient de réactif (10) se présente sous la forme d'un cylindre dont une des extrémités est fermée (11) et l'autre ouverte (12), le cylindre contenant un piston perforé (13) en matériau élastique qui scelle le récipient lui-même, et un film d'étanchéité (14) empilé à chaud sur l'extrémité ouverte du récipient;
- le piston (13) est pourvu d'un trou central réalisé au préalable au moyen de l'une aiguille d'une seringue et
- le récipient (10) est apte à être inséré sur la projection tubulaire (31) de la chambre de test du dispositif où est logée la pilule de chargement d'échantillon (30), le piston pouvant être poussé vers l'arrière par la portion tubulaire (31) jusqu'à ce qu'une pression vienne s'appliquer sur la solution de réactif en faisant coulisser le piston sur le dispositif, de telle sorte que le réactif se déplace vers la pilule de chargement d'échantillon (30) à travers le trou central du piston agissant comme une valve.

2. Dispositif de test selon la revendication 1, dans lequel ladite chambre de test comprend un boîtier tubulaire externe (21) et un élément de support interne (23) pourvu d'une série de rainures longitudinales (24), une série de bandes de test (26) adaptées pour se loger dans lesdites rainures longitudinales (24) et intercalées entre l'élément de support interne (23) et le boîtier externe (21).

3. Dispositif de test selon la revendication 2, dans lequel ledit boîtier externe (21) est pourvu d'une extrémité distale fermée du récipient de réactif dans lequel est percé un conduit d'aération, et d'une extrémité proximale opposée ouverte pour permettre l'insertion de l'élément de support interne (23) qui est pourvu de ladite projection (31) de la chambre de test.

4. Dispositif de test selon la revendication 3, dans lequel une portion tubulaire agrandie (32) se projette en saillie à partir de ladite extrémité proximale du boîtier externe (21) pour couvrir au moins partiellement le récipient de réactif (10) lorsqu'il est inséré dans la portion tubulaire (31) de l'élément de support interne (23).

5. Dispositif de test selon la revendication 2, dans lequel ledit boîtier externe (21) est pourvu d'une extrémité distale du récipient de réactif ouvert pour permettre l'insertion de l'élément de support interne (23), tandis que l'extrémité proximale opposée est pourvue de ladite projection (31).

6. Dispositif de test selon la revendication 2, dans lequel lesdites bandes de test (26) ont leur extrémité proximale (28) pliées vers l'intérieur à environ 180° pour être en contact étroit avec ladite pilule de chargement d'échantillon (30).

7. Dispositif de test selon la revendication 2, dans lequel lesdites bandes de test (26) ont leur extrémité proximale (28) pliées vers l'intérieur à environ 90° pour être en contact étroit avec un tampon absorbant (27) à son tour en contact avec ladite pilule de chargement d'échantillon (30).

8. Dispositif de test selon l'une quelconque des revendications qui précèdent, **caractérisé par le fait que** ladite pilule de chargement d'échantillon (30) comprend une enveloppe externe (30') en matériau élastique souple tel que le silicone, rempli d'un matériau absorbant, inerte pour transporter les échantillons biologiques, ou chimiquement dérivé pour enrichir de manière sélective les analytes à mesurer.

9. Dispositif de test selon la revendication 8, dans lequel ladite enveloppe externe (30') a une forme en ogive avec deux ouvertures (50) dont les extrémités opposées sont alignées le long de son axe longitudinal, lesdites ouvertures étant aptes à collecter l'échantillon à tester dans le matériau absorbant (30") ainsi qu'au passage du réactif à travers le matériau absorbant.

10. Dispositif de test selon l'une quelconque des revendications qui précèdent, dans lequel ledit récipient de réactif (10) et ladite chambre de test (20) sont fabriqués dans un matériau plastique transparent et rigide.

11. Dispositif de test selon l'une quelconque des revendications qui précèdent, dans lequel ladite chambre de test (20) a une section transversale circulaire ou polygonale.

12. Dispositif de test selon l'une quelconque des revendications qui précèdent, apte à effectuer simultanément de différents tests sur le même échantillon.

13. Procédé de fonctionnement du dispositif de test selon l'une quelconque des revendications qui précèdent, comprenant les étapes suivantes:
- insérer la pilule de chargement d'échantillon (30) imprégnée d'un échantillon de liquide dans la projection tubulaire (31) de la chambre de test (20) jusqu'à ce qu'elle soit en contact étroit avec les bandes de test (26) ou avec le tampon absorbant (27);
- retirer le film d'étanchéité (14) du récipient de réactif (10) et coupler le récipient de réactif avec la portion tubulaire (31) de la chambre de test (20);
- pousser le piston (13) vers le fond du récipient de réactif (10) à travers la portion tubulaire (31) de manière à ce que le réactif se déplace vers la pilule de chargement d'échantillon (30) à travers un trou central dans le piston (13);
- le réactif passe à travers et par-dessus la pilule de chargement d'échantillon (30) et déplace l'échantillon imprégné dans la pilule de chargement d'échantillon vers les bandes de test (26) ou le tampon absorbant (27) en contact avec celui-ci;
- les bandes de test capillaire (26) transportent le réactif de test à travers elles, et une ligne de signal de détection (35) imprimée sur les bandes de test devient visible.

14. Procédé selon la revendication 13, dans lequel on remplit de réactif ledit récipient de réactif (10) en poussant le piston (13) au fond du récipient; insérer l'aiguille d'une seringue, qui contient le réactif de test dans le piston (13) jusqu'au fond du récipient; activer la seringue de manière à ce que le réactif remplisse le récipient (10) tout en poussant le piston (13) vers l'ouverture du récipient.
